# EUROPEAN PATENT SPECIFICATION

(11) **EP 0 536 343 B1**
(45) Date of publication and mention of the grant of the patent: **20.09.1995**
(21) Application number: 92903149.0
(22) Date of filing: 28.01.1992
(51) Int. Cl.: A61K 47/48

(54) **TARGETED POLYMERIC CONJUGATES OF CYCLOSPORIN AND METHOD FOR THEIR PREPARATION**
ZIELGERICHTETE POLYMERKONJUGATE VON CYCLOSPORIN UND VERFAHREN ZU IHRER HERSTELLUNG
CONJUGUES POLYMERES CIBLES DE CYCLOSPORINE ET PROCEDE DE PREPARATION

(30) Priority: 01.02.1991 CS 251/91
(43) Date of publication of application: 14.04.1993
(73) Proprietor: GALENA, CS-747 70 Opava-Komarov (CS)
(72) Inventor: JEGOROV, Alexandr, 370 01 Ceské budejovice (CS); MATHA, Vladimir, 370 01 Ceské Budejovice (CS); ULBRICH, Karel, 140 00 Praha 4 (CS); STROHALM, Jiri, 160 00 Praha 6 (CS); RIHOVA, Blanka, 140 00 Praha 4 (CS); FORNUSEK, Lubor, 140 00 Praha 4 (CS)
(74) Representative: Tubby, David George
(86) International application number: CS9200003
(87) International publication number: WO9213569

(56) References cited:
- EP-A- 0 187 547
- WO-A-90/06763
- DE-A- 3 513 572
- JOURNAL OF CONTROLLED RELESASE Vol. 9, 1989, Amsterdam, NL, pages 21-32
- JOURNAL OF CHROMATOGRAPHY; Vol. 376, 1986 Amsterdam, NL, pages 221-233.
- JOURNAL OF CONTROLLED RELEASE Vol. 6, 1987, Amsterdam, NL, pages 315-327.
- JOURNAL OF CONTROLLED RELEASE Vol. 2, 1985, Amsterdam, NL, pages 289-310

## Description

### Technical Field

The invention relates to a targeted drug based on polymeric conjugates of cyclosporins possessing an immunosuppressive effect, which consist of an inert polymeric carrier to which cyclic undecapeptides - cyclosporins are covalently bound, along with antibodies, thus ensuring a targeted immunosuppressive effect.

### Background Art

Cyclosporin A is a cyclic undecapeptide whose structure is described by the formula: 
MeBmt-α-Abu-Sar-MeLeu-Val-MeLeu-Ala-(D)-Ala-MeLeu-MeLeu-MeVal .
1 2 3 4 5 6 7 8 9 10 11
where MeBmt is the amino acid residue of HOOC-CH(NHCH₃)-CH(OH)-CH(CH₃)-CH₂-CH=CH-CH₃, i.e. (2S,3R,4R,6E)-3-hydroxy-4-methyl-2-methylamino-6-octenoic acid.

In addition to cyclosporin A, a series of B through Z cyclosporins has been isolated. derived from cyclosporin A by substitution of the individual amino acids in the ring (Helvetica Chimica Acta 70,13,1987). These cyclosporins are a natural metabolite of some types of deuteromycets and are produced on an industrial scale by cultivating highly productive strains of fungi, e.g. of the genus Tolypocladium. A disadvantage of natural cyclosporins is that they do not contain an active group which could be employed e.g. for binding onto a carrier, with the exception of the -OH group, the chemical bond of which, however, causes the loss of activity of the derivatives. Semisynthetically, derivatives bearing a functional group can be prepared without reducing the immuno-suppressive activity of cyclosporins. An alternative, though more demanding preparation procedure is total synthesis, which was used in the preparation of some derivatives of cyclosporins containing various functional groups (European patents EP 34567 and EP 194972, Progress in the Chemistry of Organic Natural Products 50,124,1986).

In human medicine, cyclosporin A is used as an immuno-suppressive agent in transplantations; moreover, cyclosporins have antiarrhythmic and antiparasitic effects, help in the treatment of autoimmunity diseases, including AIDS, and are also being tested for other clinical indications. However, along with therapeutical effects cyclosporins also show some unfavourable side effects, due to the comparatively high therapeutical dose and consisting in their nephrotoxicity (Transplant. Proceedings 17. Suppl. 1, 101, 1985), neurotoxicity (Transplan. Proceedings 20, Suppl, 2, 374, 1988). and hepatotoxicity (Transplant. Proceedings 15, Suppl, 1,545, 1983).

### Disclosure of Invention

The disadvantage is removed by employing a procedure according to the invention, consisting in targeting the cyclosporins bound onto an inert polymeric carrier together with antibodies as determinants of the specific effect.

The advantage of said procedure consists in that, when using such targeted polymeric conjugates of cyclosporins, it is possible to use a lower quantity of the effective compound, i.e. the bound cyclosporins, compared with the use of pure cyclosporins, the main advantage being that at the same time targeting considerably reduces undesirable side effects.

The targeted polymeric conjugate of cyclosporin, wherein said conjugate as the polymeric carrier contains 40-700 polymerized monomeric units composed of 20 - 99 % mol. of biologically inert hydrophilic units, such as N-(2-hydroxypropyl)methacrylamide. N-vinylpyrrolidone or acrylic and methacrylic acid in the form of their sodium salts. advantageously N-(2-hydroxypropyl)methacrylamide, 0.1-25 % mol. units of methacryloylated amino acids or methacryloylated oligopeptides with terminally bound cyclosporin and 0 - 10 % mol. units of methacryloylated amino acids or oligopeptides onto which an antibody has been bound as the determinant of transport to the cells.

In the case of the use of N-(2-hydroxypropyl)methacrylamide, as basic monomeric units said polymeric conjugates are described by the formula:

-[-CH₂-C(CH₃)(CO-R₁)-]_{*x*}-[-CH₂-C(CH₃)(CO-R₂)-]_{*y*}-[-CH₂-C(CH₃)-(CO-R₃)-]_{*z*}-.

in which x lies in the range 20 - 99 % mol., y lies in the range 0.1 - 5 % mol., z lies in the range 0 - 10 % mol., R₁ is NH-CH₂-CH(OH)-CH₃, R₂ is amino acid or an oligopeptidic sequence with terminally bound cyclosporin, R₃ is amino acid or an oligopeptidic sequence with an antibody bound to it. If other hydrophilic monomers mentioned above are used as basic monomeric units the targeted polymeric conjugates of cyclosporin are described by an analogous formula, in which the expression for x = 20 - 99 % corresponds to the molar content of the given monomer or to the sum of the molar content of several such monomers in the case that their mixtures are used in the copolymerization.

The biologically effective component of the polymeric conjugate of cyclosporin is cyclosporin covalently bound onto the polymeric carrier by means of an amino acid or an oligopeptidic sequence. For binding onto an amino acid or oligopeptidic sequence, cyclosporins are used which possess the required immunosuppressive or other biological activities, while at the same time bearing in their structure functional groups with sufficient chemical activity, with advantage a primary or secondary amino group, hydrazine or aldehyde group. Alternatively, derivatives containing less active functional groups and activated by common procedures can be employed, e.g. using carbodiimides or bifunctional active esters. Depending on the type of the derivative of cyclosporin, its cyclic skeleton is bound either directly onto the terminal amino acid, i.e. by means of an amide bond in the case of aminocyclosporin, or through a bifunctional "shoulder", i.e. e.g. diamine attached by an amide bond to the terminal amino acid, on the one hand, and on the other, by an amine bond arising by conjugation of the aldehyde functional group of cyclosporin with the free amino group of diamine followed by reduction.

Cyclosporins and antibodies are bound onto the carrier by means of common biogenic amino acids, such as glycine, alanine, aminocaproic acid or phenylalanine, or oligopeptidic sequences thereof, e.g. GlyGly, GlyPheLeuGly, GlyLeuPheGly, LeuGlyLeuGly, GlyLeuGly, or GlyValGly. With advantage, polymeric conjugates of cyclosporin with oligopeptidic sequences between the polymeric carrier and cyclosporin are used, which can be degraded by intracellular (lysosomal) enzymes.

Proteins with a receptor expressed on the targeted tissue are used in the targeting. The targeting of the effect of polymeric conjugates of cyclosporin is achieved by binding antibodies against receptors expressed on the surface of T lymphocytes, such as anti Thy 1,2 in the mice system or pan T, i.e. anti CD3 in humans. Alternatively, targeting in order to increase the immunosuppressive effect is achieved by using antibodies aimed against the so-called CD determinants expressed on the surfaces of B or T cells, antibodies against the other receptors on cells of the immunity system, such as macrophages, mast cells and body cells which express MHC antigens of the Ist or IInd class. Targeting of the biological effect is alternatively achieved by using e.g. some hormones, carbohydrates, lectines, and in the case of the treatment of autoimmunity diseases by means of antiidiotype antibodies. Targeting is brought about by using both polyclonal and monoclonal antibodies. The latter are bound onto the carrier either specifically, i.e. without interfering with the binding site of the antibody, e.g. through the Fc part of antibody molecule, or nonspecifically, through free amino groups.

Synthesis of the polymeric conjugates of cyclosporin proceeds in three or two stages. In the former case, a polymeric carrier provided with suitable functional groups for binding cyclosporin and antibody is prepared in the first stage. In the second stage, a suitable derivative of cyclosporin is bound, and in the third stage, the antibody is bound onto the remaining functional groups. In this procedure, both cyclosporin and the antibody are bound onto carrier via oligopeptidic fragment of the same amino acid structure. In the two-stage procedure, the copolymer is prepared in the first stage from the monomer bearing cyclosporin bound to it, and the copolymerization is carried out together with methacroylamino acids or methacroyloligopeptides bearing binding sites for binding the antibody. The antibody is bound onto the free binding sites in the second step. In such two-stage procedure both cyclosporin and the antibody may be bound by means of various fragments.

In the three-stage synthesis the copolymer composed of 40 - 700 monomeric units is prepared in the first stage by radical copolymerization of 20 - 99 % mol. of monomeric units of one or several biologically inert hydrophilic monomers, with advantage monomeric units of N-(2-hydroxypropyl)methacrylamide, and 0.1 - 30 % mol. units of p-nitrophenyl ester of methacryloylated amino acid or oligopeptide. Alternatively, in the first stage the copolymer is prepared by radical copolymerization of 20 - 99 % mol. monomeric units of N-(2-hydroxypropyl)methacrylamide with 0.1 - 30 % mol. units of methacryloylated derivatives of amino acids or oligopeptides terminated with a functional group -CO-NH-(CH₂)_{*n*}-NH₂, where n = 1 through 8. A similar polymeric carrier may be prepared by aminolysis of the polymer bearing active esters with a 100-200-fold excess of e.g. ethylenediamine or hexamethylenediamine.

In the second stage the derivative of cyclosporin is bound, either via an aminolytic reaction of ester bonds of the polymeric carrier with the amino or hydrazo derivative of cyclosporin, or alternatively, condensation of the aldehyde group of cyclosporin with the amino group is used, and the bond thus formed is stabilized by reduction with sodiumborohydride, accompanied by the formation of secondary amine. Cyclosporin is bound onto the polymeric carrier in polar solvents or mixtures thereof, with advantage in dimethylsulfoxide, dimethylformamide, ethanol, or methanol. The unreacted cyclosporin is separated by repeated precipitation of the polymer into nonpolar solvents, e.g. into an acetone-ether mixture 1 : 1. An analysis of the amount of bound cyclosporin is determined following an acid hydrolysis of the polymer with 6 N hydrochloric acid and is expressed as the ratio of amino acids originating in cyclosporin and the binding amino acid or oligopeptide.

In the third stage the antibody is bound onto the remaining binding sites of the polymer. The reaction takes place in an aqueous buffer, either by an aminolytic reaction of active esters of the polymer due to free amino groups of the antibody or the aldehyde group of the antibody (obtained by the oxidation of the Fc part of its molecule with a solution of sodium periodate) reacts with free amino groups on the polymer, and the resulting bond, is stabilized by means of a suitable reducing agent, e.g. sodium borohydride or sodium cyanoborohydride.

In the two-stage synthesis of polymeric conjugates of cyclosporin the copolymer consisting of 40-700 monomeric units is prepared in the first step by radical copolymerization of 20 - 99 % mol. monomeric units of one or several biologically inert hydrophilic monomers, with advantage monomeric units of N-(2-hydroxypropyl)methacrylamide, with 0.1 - 25 % mol. units of methacroyl oligopeptidyl cyclosporin and with 0.1 - 10 % mol. units of the methacryloylated derivative of amino acid or oligopeptide terminated with the NH-(CH₂)_{*n*}NH₂ group, n = 1 through 8, or with p-nitrophenyl ester: derivatives of diglycine are advantageously used. In the second stage the antibody is bound onto the polymer by employing a procedure similar to that described for the three-stage synthesis, i.e. by aminolysis of p-nitrophenyl ester or by a reaction between the oxidized Fc part of antibody molecule with free amino groups of the polymer.

The invention is explained below on examples, by which of course its extent is neither limited nor exhausted.

### Examples

### Example 1:

By employing the radical precipitation copolymerization of 3.0 g N-(2-hydroxypropyl)methacrylamide with 0.57 g methacryloylated p-nitrophenyl ester of glycylphenylalanylleucylglycine in 32.6 ml of acetone with 0.18 g azo-bis-isobutyronitrile as initiator at 50 °C (the polymerization mixture was bubbled through with nitrogen, sealed in a glass ampoule, polymerization time 24 h), a polymer with the molar mass 21000 was prepared (R₂ = GlyPheLeuGlyONp), bearing in its side chains 4 % mol. oligopeptidic sequences terminated with a reactive p-nitrophenyl ester, x = 96, y = 4.

### Example 2:

0.19 g of the derivative of cyclosporin A, which instead of MeBmt contains amino acid having the formula HOOC-CH(NHCH₃)-CH(OH)-CH(CH₃)-(CH₂)₅NHNH₂ [CyA-CH₂NHNH₂], was dissolved in 2.1 ml of dimethylsulfoxide and the solution thus obtained was added to a solution of 1.7 g of the polymeric carrier prepared according to Example 1 and dissolved in 10 ml of dimethylsulfoxide. After 17 h the reaction was completed by precipitating the polymer into 600 ml of the acetone-ether mixture 1 : 1 v/v, and the product was repurified by double reprecipitation from methanol into the same mixture, R₂ = GlyPheLeuGlyNHNH-CH₂-CyA. The content of the bound cyclosporin was found to be 9.5 % by mass by an analysis of amino acids in the polymer hydrolyzate.

### Example 3:

By employing the same procedure as in Example 2, a derivative of cyclosporin G containing instead of MeBmt an amino acid having the formula HOOC-CH(NHCH₃)-CH(OH)-CH(CH₃)-(CH₂)₃-CH(CH₃)NH-(CH₂)₂-NH₂ [i-CyG-CH₂-NH-(CH₂)₂-NH₂] was bound onto the polymeric precursor prepared as in Example 1. Instead of 0.19 g CyA-CH₂NHNH₂, 0.19 g of i-CyG-CH₂NH(CH₂)₂-NH₂, R₂ = GlyPheLeuGlyNH(CH₂)₂-NH-(CH₂)-(i-CyG) was used in this case. The content of bound cyclosporin as determined by an analysis of amino acids in the polymer hydrolyzate was 10.1 % by mass.

### Example 4:

1.0 g of the polymeric carrier prepared as in Example 1 was dissolved in 4 ml of methanol, and 2 ml of freshly distilled ethylenediamine was added. The mixture was stirred at room temperature 4 h and the excess of amine was evaporated in vacuo. The polymer was dissolved in 5.5 ml of methanol and repurified twice by repeated precipitation after 300 ml of the acetone-diethyl ether mixture (1:1 v/v) had been added. The content of free amino groups in the polymer was 3.8 % mol., R₂ = GlyPheLeuGlyNH(CH₂)₂NH₂. 1.0 g of the polymeric carrier thus prepared was dissolved in 10 ml of methanol and a solution of 0.11 g of the derivative of cyclosporin A in 1 ml of methanol was added, with an amino acid having the formula HOOC-CH(NHCH₃)-CH(OH)-CH(CH₃)-(CH₂)₄CHO [CyA-CHO] instead of MeBmt. After 4h the reaction mixture was cooled to 4 °C and 20 mg sodium borohydride was added. After another 90 min. methanol was evaporated in vacuo. the polymer was dissolved in 20 ml water and repurified by gel chromatography on a column packed with Sephadex® G-25, using water as eluent. The repurified polymer was lyophilized. R₂ = GlyPheLeuGlyNH(CH₂)₂NH-CH₂-CyA. The content of bound cyclosporin determined by an analysis of amino acids in the polymer hydrolyzate was 9.2 % by mass.

### Example 5:

By radical precipitation copolymerization of 3.0 g N-(2-hydroxypropyl)methacrylamide with 1.2 g methacryloylated p-nitrophenyl ester of glycylphenylalanylleucylglycine in 32.6 ml of acetone with 0.18 g azo-bis-isobutyronitrile as the initiator at 50 °C (the polymerization mixture had been bubbled through with nitrogen, sealed in a glass ampoule, polymerization time 24 h), a polymer having molar mass 18000. R₂ = GlyPheLeuGlyONp was prepared, bearing in its side chains 9 % mol. oligopeptidic sequences terminated with reactive p-nitrophenyl ester, x = 91, y = 9.

### Example 6:

Cyclosporin was bound according to Example 2 with a polymeric carrier prepared as in Example 5. The analysis revealed that the polymer contained 9.6 % by mass cyclosporin and approx. 4 % mol. of unreacted ONp groups, R₂ = GlyPheLeuGly-NHNH-CH₂-CyA, R₃ = GlyPheLeuGlyONp, x = 91, y approx. 2, z approx. 4. 200 mg of the polymer thus prepared was dissolved in 1.6 ml of 0.15 M sodium chloride, 1.3 ml of the borate buffer pH 8 was added, and the solution was cooled to 4 °C. A 5 - 6 % solution of 67 mg of antibodies in the PBS buffer was added. and for two hours the pH of the reaction mixture was maintained at 7.5 by using a saturated solution of sodium tetraborate. After that, pH of the reaction mixture was raised to 9.0 by adding sodium tetraborate and the reaction continued for another three hours. After that, the polymer thus obtained, in which R₃ was now GlyPheLeuGly-NHPr. NHPr being an antibody bound by the amide bond, was purified by gel chromatography on a column packed with Sephadex® G-15 and washed with a buffer having pH 7.2. The solution was used for biological testing. The conjugate was characterized by gel chromatography on a column packed with Sepharose® 4B and 6B, 1 : 1 by mass, and electrophoretically. Mass balance showed that the sample contains 25 % by mass of antibodies and 7.2 % by mass of bound cyclosporin.

### Example 7:

1.5 g of the polymeric carrier prepared as in Example 5 was dissolved in 6 ml of methanol and 6 ml of ethylenediamine was added with vigorous stirring After stirring for 4 h the unreacted ethylenediamine and methanol were evaporated in a vacuum evaporator. The dry residue was dissolved in 11 ml of methanol and repurified twice by repeated precipitation after the addition of 400 ml of the acetone-diethyl ether mixture (1 : 1 v/v). To 1 g of the polymer thus modified, in which R₂ = GlyPheLeuGlyNH(CH₂)₂NH₂, x = 91, y = 9, dissolved in 10 ml of methanol, a solution of 200 ml of a derivative of cyclosporin A dissolved in 2 ml of methanol was added, with an amino acid having the formula HOOC-CH(NHCH₃)-CH(CH₃)-(CH₂)₄-CHO [CyA-CHO] instead of MeBmt. After 4 h the reaction mixture was cooled to 0 °C, and 30 mg of sodium borohydride was added. The reaction was carried out at this temperature for one hour. Methanol was evaporated in a vaccum evaporator, the dry residue was dissolved in 20 ml of water and repurified by gel chromatography on a column 4 x 40 cm packed with Sephadex® G-25. An amino acids analysis showed that the polymer thus prepared, in which R₂ = GlyPheLeuGlyNH(CH₂)₂NH-CH₂CyA contained 17 % by mass of bound cyclosporin.

700 mg of antibodies was dissolved in 0.1 M of the acetate buffer, pH 4.0. containing 0.15 M of sodium chloride to a final concentration of 21 mg/ml. To 20 ml of the solution of antibodies, 28 ml 0.2 M of sodium periodate in the same buffer was added at 0 °C. and the mixture was stirred 90 min. After that the oxidized antibodies were separated from salts on a column packed with Sephadex® G-25, and their solution was concentrated by ultrafiltration to a concentration of 20 mg/ml.

1 g of the polymer with bound cyclosporin was dissolved in 7 ml of 0.1 M acetate buffer, pH 4.0, containing 0.15 M of sodium chloride. On cooling to 4 °C, a solution of 330 mg oxidized antibodies in 16.5 ml of acetate buffer was added. The pH of the solution was adjusted to 8.0 by using a saturated solution of sodium tetraborate, and the stirring continued for another 16 h. At the same temperature 17 mg sodium cyanoborohydride was added, and after stirring for 2 h the product was purified by gel chromatography on a column 4 x 40 cm packed with Sephadex® G-25 and eluted with a phosphate buffer, pH 7.2. The conjugate was characterized by gel chromatography and electrophoretically, and the concentration of the conjugate in solution was determined from the content of dry residue after lyophilization of the desalinated sample. Mass balance revealed that the sample contains 25 % by mass of antibodies and 12.8 % by mass of bound cyclosporin, the concentration being 20 mg/ml. where R₂ = GlyPheLeuGlyNH(CH₂)2NH-CH₂CyA, R₃ = GlyPheLeuGlyNH(CH₂)₂-NH-CH₂-Pr. CH₂-Pr being an antibody bound through the oxidized Fc part of the molecule.

### Example 8:

The radical precipitation copolymerization of 200 mg Ma-GlyPheLeuGly-NH-(CH₂)₂-NH-CH₂-CyA, 60 mg Ma-GlyGly-NH-(CH₂)₂-NH₂ and 800 mg N-(2-hydroxypropyl)methacrylamide, where Ma is methacryloyl, was carried out in acetone with 0.8 % by mass azobisisobutyronitrile, at 50 °C, in nitrogen atmosphere, in a sealed glass ampoule at the total concentration 12 % by mass of monomers in the polymerization mixture, for 24 h. Acetone was evaporated in a vacuum evaporator, the dry residue was dissolved in water and purified by gel chromatography on a column 4 x 40 cm packed with Sephadex® G-25 using water as eluent, and lyophilized. Binding of the antibodies and purification of the conjugate were performed as in Example 7. The conjugate contained 10 % by mass cyclosporin and 25 % by mass antibodies, R₂ = GlyPheLeuGly-NH-(CH₂)₂-NH-CH₂-CyA, R₃ = GlyGly-NH-(CH₂)₂-NH-CH₂-Pr, where CH₂-Pr is the antibody bound through the oxidized Fc part of the molecule.

### Example 9:

From fresh human heparinized blood human lymphocytes were isolated and placed in the tissue culture. Their sensitivity to the action of cyclosporin, targeted with the CD antibody and prepared as in Example 6, in which the rabbit anti CD3 IgG was bound as the antibody, was tested by the inhibition of proliferation induced in the culture by means of the cell mitogen Concanavaline A and tested as the incorporation of ³H-thymidine. It was found that the targeted cyclosporin distinctly reduces the proliferation of T cells up to the concentration of 100 ng/ml. The result proves that the binding of cyclosporin to the polymeric carrier does not reduce its pharmacological activity.

### Example 10:

The inhibitive effect of anti Thy 1,2 caused by the cyclosporin targeted with the anti Thy 1,2 antibody prepared as in Example 7, where the rabbit anti Thy 1.2 IgG was bound as the antibody, was tested in vitro using a mixed culture of mice splenocytes. Splenocytes H-2a were the corresponding cells, splenocytes H-2b were the stimulating cells. It was found that the proliferation of the H-2a cells induced by the presence of foreign cells H-2b was reduced to a concentration of 10-100 ng/ml. The result shows that the pharmacological activity of the targeted cyclosporin is not decreased by binding to the polymeric carrier.

### Example 11:

Nephrotoxicity of the targeted cyclosporin prepared as in Examples 6,7 was tested on males of the Wistar strain. A dose corresponding to 50 mg of cyclosporin bound in the targeted conjugated recalculated to one kg of weight was administered intraperitoneally to the test group for 10 days, while a dose of 50 mg per one kg of free cyclosporin A was administered to the control group. After ten days the kidneys, thymus, liver, and spleen were subjected to histological analysis. It was found that, while free cyclosporin A administered in such dose causes grave damage to the kidneys, reflected as a toxic tubular lesion of proximal tubuli, no pathological changes were caused in the kidneys by the targeted conjugate of cyclosporin.

### Industrial application

The invention can be employed in medicine particulary in transplantations of organs, when it is necessary to suppress temporarily some immunity components and make possible the acception of the transplanted organ, and in those indications where, by affecting cells of the immunity system, pathological states can be treated, as e.g. in the treatment of autoimmunity diseases.

## Claims

1. Polymeric conjugates of cyclosporin characterized in that they as polymeric carriers contain 40 - 700 polymerized monomeric units composed of 20 - 99 % mol. biologically inert hydrophilic units, such as N-(2-hydroxypropyl)methacrylamide, N-vinylpyrrolidone or acrylic and methacrylic acid in the form of sodium salts, with advantage N-(2-hydroxypropyl)methacrylamide, 0.1 - 25 % mol. units of methacryloylated amino acids or methacryloylated oligopeptides with terminally bound cyclosporin and 0 - 10 % mol. units of methacryloylated amino acids or oligopeptides, with an antibody bound onto them as the determinant of transport to the targeted cells.

2. Polymeric conjugates of cyclosporin according to claim 1, wherein cyclosporin bearing aldehyde, hydrazine, or the primary amino group as the functional group are used in binding onto the carrier.

3. Targeted polymeric conjugates of cyclosporin according to claim 1, wherein anti-bodies used in the targeting are bound by means of free amino groups or of the oxidized Fc part of the antibody molecule.

4. Targeted polymeric conjugates of cyclosporin according to any preceding claim, wherein the antibodies are targeted against receptors expressed on the surface of T lymphocytes.

5. Targeted polymeric conjugates of cyclosporin according to any preceding claim, wherein the targeting of the biological effect is achieved by using of hormones, carbohydrates, lectines or antiidiotype antibodies.

6. A manner of manufacture of polymeric cojugates of cyclosporin according to any preceding claim, characterized by copolymerization of 20 - 99 % mol. monomeric units of one or several biologically inert hydrophilic monomers, with advantage monomeric units of N-(2-hydroxypropyl)methacrylamide, with 0.1 - 25 % mol. units of methacroyl oligopeptidyl cyclosporin and with 0.1 - 10 % mol. units of the methacryloylated derivative of amino acid or oligopeptide terminated with the NH-(CH₂)_{*n*}NH₂ group, n = 1 through 8, or with p-nitrophenyl ester, and then in the second stage the antibody is bound onto the polymer by aminolysis of p-nitrophenyl ester or by a reaction between the oxidized Fc part of antibody molecule with free amino groups of the polymer.

7. A manner of manufacture of polymeric conjugates of cyclosporin according to any of claims 1 to 5, characterized by copolymerization of 20 - 99 % mol. of monomeric units of one or several biologically inert hydrophilic monomers, with advantage monomeric units of N-(2-hydroxypropyl)methacrylamide, and 0.1 - 30 % mol. units of p-nitrophenyl ester of methacryloylated amino acid or oligopeptide or alternatively, in the first stage the copolymer is prepared by radical copolymerization of 20 - 99 % mol. monomeric units of N-(2-hydroxypropyl)methacrylamide with 0.1 - 30 % mol. units of methacryloylated derivatives of amino acids or oligopeptides terminated with a functional group -CO-NH-(CH₂)_{*n*}-NH₂, where n = 1 through 8, then in the second stage the derivative of cyclosporin is bound, either via an aminolytic reaction of ester bonds of the polymeric carrier with the amino or hydrazo derivative of cyclosporin, or alternatively, condensation of the aldehyde group of cyclosprorin with the amino group is used, and the bond thus formed is stabilized by reduction with sodium borohydride, accompanied by the formation of secondary amine, finally in the third stage the antibody is bound onto the remaining binding sites of the polymer.

## Patentansprüche

1. Polymerkonjugate von Cyclosporin, dadurch gekennzeichnet, daß sie als polymere Träger 40-700 polymerisierte Monomereinheiten enthalten, zusammengesetzt aus 20-99 Mol-% biologisch inaktiven hydrophilen Einheiten, wie z.B. N-(2-Hydroxypropyl)methacrylamid, N-Vinylpyrrolidon oder Acryl- und Methacrylsäure in Form von Natriumsalzen, vorzugsweise aus N-(2-Hydroxypropyl)methacrylamid, 0,1-25 Mol-% methacryloylierten Aminosäure- oder methacryloylierten Oligopeptid-Einheiten mit terminal gebundenem Cyclosporin und 0-10 Mol-% methacryloylierten Aminosäure- oder Oligopeptid-Einheiten mit einem daran gebundenen Antikörper als Determinant für den Transport zu den Zielzellen.

2. Polymerkonjugate von Cyclosporin nach Anspruch 1, wobei ein cyclosporintragendes Aldehyd, Hydrazin oder die primäre Aminogruppe als funktionelle Gruppe zur Bindung an den Träger verwendet werden.

3. Zielgerichtete Polymerkonjugate von Cyclosporin nach Anspruch 1, wobei bei der Zielorientierung verwendete Antikörper mit Hilfe freier Aminogruppen oder des oxidierten Fc-Teils des Antikörpermoleküls gebunden werden.

4. Zielgerichtete Polymerkonjugate von Cyclosporin nach einem der vorhergehenden Ansprüche, wobei die Antikörper gegen Rezeptoren gerichtet sind, die an der Oberfläche von T-Lymphozyten exprimiert sind.

5. Zielgerichtete Polymerkonjugate von Cyclosporin nach einem der vorhergehenden Ansprüche, wobei die Zielorientierung der biologischen Wirkung durch Verwendung von Hormonen, Kohlenwasserstoffen, Lectinen oder antiidiotypischen Antikörpern erzielt wird.

6. Verfahren zur Herstellung von Polymerkonjugaten von Cyclosporin nach einem der vorhergehenden Ansprüche, gekennzeichnet durch eine Copolymerisation von 20-99 Mol-% Monomereinheiten eines oder mehrerer biologisch inaktiver hydrophiler Monomere, vorzugsweise Monomereinheiten von N-(2-Hydroxypropyl)methacrylamid, mit 0,1-25 Mol-% Einheiten von Methacroyloligopeptidylcyclosporin und mit 0,1-10 Mol-% Einheiten des methacryloylierten Derivats einer Aminosäure oder eines Oligopeptids, abgeschlossen mit der NH-(CH₂)ₙNH₂-Gruppe, n = 1 bis 8, oder mit p-Nitrophenylester, wobei dann in der zweiten Stufe der Antikörper durch Aminolyse von p-Nitrophenylester oder durch eine Reaktion zwischen dem oxidierten Fc-Teil des Antikörpermoleküls mit freien Aminogruppen des Polymers an das Polymer gebunden wird.

7. Verfahren zur Herstellung von Polymerkonjugaten von Cyclosporin nach einem der Ansprüche 1 bis 5, gekennzeichnet durch eine Copolymerisation von 20-99 Mol-% Monomereinheiten eines oder mehrerer biologisch inaktiver hydrophiler Monomere, vorzugsweise Monomereinheiten von N-(2-Hydroxypropyl)methacrylamid, und 0,1-30 Mol-% Einheiten von p-Nitrophenylester von methacryloylierter Aminosäure oder Oligopeptid, oder alternativ durch Herstellung des Copolymers in der ersten Stufe mittels Radikalcopolymerisatlon von 20-99 Mol-% Monomereinheiten von N-(2-Hydroxypropyl)methacrylamid mit 0,1-30 Mol-% Einheiten von methacryloylierten Derivaten von Aminosäuren oder Oligopeptiden, die durch eine funktionelle -CO-NH-(CH₂)ₙ-NH₂-Gruppe abgeschlossen sind, mit n = 1 bis 8, wobei dann in der zweiten Stufe das Cyclosporin-Derivat entweder mittels einer aminolytischen Reaktion von Esterbindungen des polymeren Trägers mit dem Amino- oder Hydrazo-Derivat von Cyclosporin gebunden oder alternativ mittels eine Kondensation der Aldehydgruppe von Cyclosporin mit der Aminogruppe verwendet wird, und wobei die so gebildete Bindung durch Reduktion mit Natriumborhydrid stabilisiert wird, begleitet von der Bildung eines sekundären Amins, und wobei schließlich in der dritten Stufe der Antikörper an die verbleibenden Bindungsstellen des Polymers gebunden wird.

## Revendications

1. Conjugués polymères de cyclosporine caractérisés en ce qu'ils contiennent, comme véhicules polymères, 40-700 unités monomères polymérisées composées de 20-99% mol. d'unités hydrophiles biologiquement inertes, telles que le N-(hydroxy-2-propyl)méthacrylamide, la N-vinylpyrrolidone ou l 'acide acrylique et méthacrylique sous forme de sels de sodium, avec de préférence le N-(hydroxy-2-propyl)méthacrylamide, 0,1-25% mol. d'unités d'acides aminés méthacryloylés ou d'oligopeptides méthacryloylés avec une cyclosporine liée en position terminale et 0-10% mol. d'unités d'acides aminés ou d'oligopeptides méthacryloylés, avec un anticorps qui y est lié en tant que déterminant du transport vers les cellules ciblées.

2. Conjugués polymères de cyclosporine selon la revendication 1, dans lesquels des groupes aldéhyde, hydrazine, ou amine primaire, comme groupes fonctionnels, portant des cyclosporines, sont utilisées pour la liaison au véhicule.

3. Conjugués polymères ciblés de cyclosporine selon la revendication 1, dans lesquels les anticorps utilisés dans le ciblage sont liés au moyen de groupes amine libres ou de la partie Fc oxydée de la molécule d'anticorps.

4. Conjugués polymères ciblés de cyclosporine selon l 'une quelconque des revendications précédentes, dans lesquels les anticorps sont ciblés contre des récepteurs exprimés à la surface des lymphocytes T.

5. Conjugués polymères ciblés de cyclosporine selon l 'une quelconque des revendications précédentes, dans lesquels le ciblage de l'effet biologique est effectué en utilisant des hormones, des hydrates de carbone, des lectines ou des anticorps antiidiotypes.

6. Procédé de fabrication de conjugués polymères de cyclosporine selon l'une quelconque des revendications précédentes, caractérisé par une copolymérisation de 20-99% mol. d'unités monomères d'un ou plusieurs monomères hydrophiles biologiquement inertes, avec de préférence des unités monomères de N-(hydroxy-2-propyl)méthacrylamide, avec 0,1-25% mol. d'unités de méthacroyl-oligopeptidyl-cyclosporine et avec 0,1-10% mol. d'unités du dérivé méthacryloylé d'un acide aminé ou d'un oligopeptide terminé par le groupe NH-(CH₂)ₙNH₂, n=1 à 8 ou par un p-nitrophényl ester, et ensuite dans la deuxième étape, l 'anticorps est lié au polymère par une aminolyse du p-nitrophényle ester ou par un réaction entre la partie Fc oxydée de la molécule d'anticorps avec des groupes amine libres du polymère.

7. Procédé de fabrication de conjugués polymères de cyclosporine selon l'une quelconque des revendications 1 à 5 caractérisé par une copolymérisation de 20-99% mol. d'unités monomères d'un ou plusieurs monomères hydrophiles biologiquement inertes, avec de préférence des unités monomères de N-(hydroxy-2-propyl)méthacrylamide, et 0,1-30% mol. d'unités du p-nitrophényl ester de l'acide aminé ou de l'oligopeptide méthacryloylé, ou alternativement, dans la première étape, le copolymère est préparé par une copolymérisation radicalaire de 20-99% mol. d'unités monomères de N-(hydroxy-2-propyl)méthacrylamide avec 0,1-30% mol. d'unités de dérivés méthacryloylés d'acides aminés ou d'oligopeptides terminés par un groupe fonctionnel -CO-NH-(CH₂)ₙ-NH₂, ou n=1 à 8, ensuite, dons la deuxième étape, le dérivé ce cyclosporine est lié, soit par une réaction aminolytique des liaisons ester du véhicule polymère avec un dérivé amino- ou hydrazo- de la cyclosporine, soit, alternativement, la condensation du groupe aldéhyde de la cyclosporine avec le groupe amine est utilisée et la liaison ainsi formée est stabilisée par réduction avec du borohydrure de sodium, accompagnée par la formation d'une amine secondaire, enfin dons la troisième étape, l'anticorps est lié sur les sites de liaison restants du polymère.
